# EUROPÄISCHE PATENTSCHRIFT

(11) **EP 0 729 739 B1**
(45) Veröffentlichungstag und Bekanntmachung des Hinweises auf die Patenterteilung: **28.04.2004**
(21) Anmeldenummer: 96102503.8
(22) Anmeldetag: 20.02.1996
(51) Int. Cl.: A61K 6/04

(54) **Angiessbare Konstruktionselemente für die Dentaltechnik**
Dental construction element for use in a casting-on process
Elément de construction dentaire utilisable dans un procédé de coulée

(30) Priorität: 25.02.1995 DE 19506681
(43) Veröffentlichungstag der Anmeldung: 04.09.1996
(73) Patentinhaber: DeguDent GmbH, 63457 Hanau (DE)
(72) Erfinder: Kempf, Bernd, Dr., 63839 Kleinwallstadt (DE); Ringelstein, Hans-Martin, 60358 Frankfurt/M. (DE); Völcker, Alexander, Dr., 63517 Rodenbach (DE); Birkholz, Ulrich, 63457 Hanau (DE)
(74) Vertreter: Stoffregen, Hans-Herbert, Dr. Dipl.-Phys.

(56) Entgegenhaltungen:
- EP-A- 0 464 951
- EP-A- 0 475 528
- DE-A- 4 306 542

## Beschreibung

Die Erfindung betrifft angießbare Konstruktionselemente für die Dentaltechnik aus Edelmetallegierungen mit Gehalten an oxidbildenden Unedelmetallen.

Bei der Herstellung von herausnehmbaren Zahnersatz wird häufig eine geeignete Verbindungstechnik benötigt, die eine Verbindung des Zahnersatzes mit dem Restgebiß oder auch mit Dentalimplantaten ermöglicht. Zur Verwendung kommen dafür zunehmend konfektionierte Teile, sogenannte Konstruktionselemente, wie Geschiebe, Brückenanker oder Wurzelstifte. Die Teile werden an den metallischen Zahnersatz angelötet beziehungsweise angegossen. Die Technik des Angießens wird dabei zunehmend gegenüber dem Löten bevorzugt, da man damit die Einbringung einer weiteren Legierung (Lot) vermeiden kann, was sich günstig in bezug auf die Korrosionsfestigkeit auswirkt.

Die Verbindung entsteht in beiden Fällen durch metallurgische Vorgänge, indem zwischen einer flüssigen und einer festen metallischen Phase eine Legierungsbildung stattfindet. Diese kann jedoch auf eine nur wenige Atomlagen ausgedehnte Reaktionszone beschränkt sein. Im Falle der Angußtechnik übernimmt die angegossene Legierung selbst die Rolle des Lotes.

Um beim Angießen einen wirklichen Materialverbund zwischen dem Konstruktionselement und der angegossenen Legierung zu erhalten, darf es bei der Vorwärmung des Konstruktionselements zu keiner Bildung von Deckschichten in Form von Oxiden oder anderen Verbindungen kommen. Es kamen daher bisher lediglich Legierungen zum Einsatz, die keine oxidierbaren Bestandteile, das heißt keine Nichtedelmetalle enthalten. Die so eingesetzten Legierungen müssen zusätzlich ausreichende Festigkeiten aufweisen und einen Schmelzbereich besitzen, der deutlich über dem der anzugießenden Legierung liegt, damit das Konstruktionselement beim Angußvorgang nicht angeschmolzen wird.

Zum Einsatz kommen daher Goldbasislegierungen mit hohen Gehalten an Palladium und Platin. Typische Legierungen weisen Zusammensetzungen mit ca. 60 Gew.% Gold, ca. 25 Gew.% Platin und ca. 15 Gew.% Palladium auf. Auch Legierungen mit ca. 80 Gew.% Platin und 20 Gew.% Iridium finden Verwendung. In der DE-OS 35 42 641 wird beispielsweise eine Legierung beschrieben, die aus 40 bis 70 % Platin, 10 bis 40 % Palladium, 5 bis 20 % Iridium und 0,5 bis 10 % Gold besteht.

Alle bisher verwendeten Legierungen für angießbare Konstruktionselemente haben aufgrund ihrer hohen Anteile an Platingruppenmetallen eine weiße Farbe und einen sehr niedrigen Wärmeausdehnungskoeffizienten (WAK). Bei den konventionellen Aufbrennlegierungen mit WAK-Werten von ca. 14·10⁻⁶/°K war dies noch akzeptabel beziehungsweise nicht vermeidbar.

Seit kurzem sind neue, goldgelbe Aufbrennlegierungen mit einem WAK von ca. 17·10⁻⁶/°C auf dem Markt, die mit einer speziellen, niedriger schmelzenden Dentalkeramik verblendet werden können. Für diese Legierung sind die derzeit zur Verfügung stehenden angießbaren Legierungen nicht geeignet. Neben dem deutlichen Unterschied in der Legierungsfarbe ist insbesondere der wesentlich größere Unterschied im WAK zwischen den Legierungen von Nachteil. Die große WAK-Differenz führt leicht zu Sprungbildungen in der Dentalkeramik, die einen an die gelben Legierungen angepaßten und damit höheren WAK-Wert aufweist, als die konventionellen Dentalkeramiken.

Für diese goldgelben Aufbrennlegierungen sind Konstruktionselemente aus gelben Legierungen erwünscht, damit sich diese vom Grundmaterial nicht farblich abheben. Diese gelben Legierungen sind bisher alle mit Nichtedelmetallen legiert und damit nicht angußfähig. Sie sind in der Regel auf der Basis Gold-Platin-Silber-Kupfer aufgebaut und verdanken ihre mechanische Festigkeit zum großen Teil der Silber-Kupfer-Mischungslücke. Durch den dadurch bedingten relativ hohen Kupfer-Anteil ist potentiell eine Verfärbungsneigung speziell bei Vorliegen einer Spaltkorrosionssituation gegeben. Außerdem bildet das Kupfer beim Erhitzen des Konstruktionselements an der Luft Oxide, die einem Anguß hinderlich sind.

Aus der EP 0 464 951 A1 ist ein Verfahren zur Herstellung eines Zahnersatzes sowie eines Gerüstes dieses bekannt. Zur Herstellung des Gerüstes wird eine Mischung aus Dentalmetallpulver oder thermoplastischem Polymer auf ein Modell aus einem porösen feuerfesten Material aufgebracht, erhitzt und sodann gesintert. Zur Vermeidung einer Oxidbildung auf den Dentalmetallpulverpartikeln sind diese mit Metallschichten aus z.B. Edelmetall versehen.

Aus der EP 0 475 528 A2 ist ein Gerüst eines Zahnersatzes bekannt, das mit einer Keramik verblendbar ist, die einen Ausdehnungskoeffizienten > 14,5 µm/m. °C aufweist.

Es war daher Aufgabe der vorliegenden Erfindung, angießbare Konstruktionselemente für die Dentaltechnik aus Edelmetallegierungen mit Gehalten an oxidbildenden Unedelmetallen zu entwickeln, die eine gelbe Goldfarbe zeigen, äußerst korrosionsbeständig und im Wärmeausdehnungskoeffizienten der Angußlegierung angepasst sind und vor allem an der Angußstelle keine Oxidschichten ausbilden.

Diese Aufgabe wird erfindungsgemäß dadurch gelöst, daß die Konstruktionselemente aus einer titanhaltigen Goldlegierung bestehen und mit einer Goldschicht überzogen sind.

Vorzugsweise enthält die Goldlegierung 0,5 bis 4 Gew.% Titan. Außerdem kann sie noch bis zu 40 Gew.% Silber, bis zu 10 Gew.% Palladium und/oder Platin und bis zu 3 Gew.% eines oder mehrere der Unedelmetalle Kupfer, Zink, Indium, Zinn, Niob, Tantal, Wolfram, Germanium und Eisen enthalten.

Weiterhin ist es von Vorteil, wenn nur die Angußstelle des Konstruktionselements eine partielle Goldschicht trägt, deren Dicke vorzugsweise zwischen 3 und 100 µm liegt.

Zur Aufbringung der Goldschicht sind im Prinzip eine Reihe von Verfahren geeignet, wobei das Verfahren und die aufzubringende Mindestschichtdicke immer optimal an die jeweilige Legierung angepaßt werden müssen. Geeignete Verfahren zur Schichtherstellung sind das galvanische Abscheiden, das Walzplattieren oder auch das Herausbeizen oxidierbarer Legierungsbestandteile durch ein geeignetes Beizmittel.

Es hat sich gezeigt, daß bei Konstruktionselementen aus Gold mit 0,5 bis 4 Gew.% Titan, auf die Schichten aus reinem Gold aufgebracht sind, eine Oxidation der Konstruktionselemente zuverlässig verhindert wird. Bereits Goldschichten mit einer Dicke von 3 µm sind für diesen Zweck ausreichend. Während der beim Anguß stattfindenden Wärmebehandlung kommt es außerdem zu einer Aufhärtung der Goldschicht, so daß die Materialverbunde eine hohe Verbindungsfestigkeit besitzen.

Nachfolgende Beispiele sollen die Erfindung näher erläutern:
1. Aus einer Gold-Titan-Legierung mit einem Anteil von 2 Gew.% Titan und einem Schmelzintervall von 1120-1090°C und einem WAK von 15,0·10⁻⁶ wird ein Wurzelstift hergestellt, der anschließend galvanisch mit einer Goldschicht von ca. 20 µm Stärke beschichtet wird. An diesen Wurzelstift wird eine gelbe niedrigschmelzende Legierung der Zusammensetzung: Au 73.8, Pt 9, Ag 9.2, Cu 4.4, Zn 2, In 1.5, Ir 0.1, mit einem Schmelzinterväll von 990-900° C und einem WAK von ca. 17·10⁻⁶ angegossen. Es kommt zu einem ausgezeichneten Materialverbund. Bei der Nachbearbeitung wird der Zahnersatz noch so weit angeschliffen, daß das Wurzelstiftmaterial freigelegt wird. Trotzdem ist diese Stelle farblich kaum zu erkennen. Auch die nachfolgende keramische Verblendung führt zu keinerlei Problemen.
2. Mittels Walzplattieren wird eine 40 µm dicke Goldfolie auf eine gelbe Legierung der Zusammensetzung Au 95.1, Cu 3, Ti 1.9 aufgebracht. Aus dem Halbzeug werden anschließend mit spanabhebenden Verfahren Konstruktionselemente herausgearbeitet, die in den vorgesehenen Angußbereichen ihre oxidationsschützende Schicht aus Gold behalten. An diese Konstruktionselemente wird eine niedrigschmelzende gelbe Legierung angegossen. Die beiden Legierungen heben sich farblich kaum voneinander ab und lassen sich ohne Probleme mit einer niedrigschmelzenden Keramik bei 800° C verblenden.

## Patentansprüche

1. Angießbare Konstruktionselemente für die Dentaltechnik aus Edelmetallegierungen mit Gehalten an oxidbildenden Unedelmetallen,
**dadurch gekennzeichnet,**
**daß** die Konstruktionselemente aus einer titanhaltigen Goldlegierung bestehen und mit einer Goldschicht überzogen sind.

2. Konstruktionselemente nach Anspruch 1,
**dadurch gekennzeichnet,**
**daß** sie aus einer Goldlegierung mit 0,5 bis 4 Gew.% Titan bestehen.

3. Konstruktionselemente nach Anspruch 1 oder 2,
**dadurch gekennzeichnet,**
**daß** nur die Angußstellen mit einer Goldschicht überzogen sind.

4. Konstruktionselemente nach Anspruch 1 bis 3,
**dadurch gekennzeichnet,**
**daß** die Goldschicht eine Dicke von 3 bis 100 µm aufweist.

## Claims

1. Construction elements for the dental technology capable of being cast on, made of oxide-forming base metals,
**characterized in**
**that** the construction elements consist of a titanium-containing gold alloy and are covered with a gold film.

2. Construction elements according to claim 1,
**characterized in**
**that** they consist of a gold alloy with 0.5 to 4 wt.% titanium.

3. Construction elements according to claim 1 or 2,
**characterized in**
**that** only the places of casting-on are covered with a gold film.

4. Construction elements according to claim 1 to 3,
**characterized in**
**that** the gold film has a thickness of 3 to 100 µm.

## Revendications

1. Eléments de construction rapportés par coulée destinés à la technique dentaire, en alliage de métaux nobles contenant des métaux communs formateurs d'oxydes,
**caractérisés en ce que**
les éléments de construction sont constitués par un alliage d'or contenant du titane et recouverts d'une couche d'or.

2. Eléments de construction selon la revendication 1,
**caractérisés en ce qu'**
ils sont constitués par un alliage d'or contenant 0,5 à 4 % en poids de titane.

3. Eléments de construction selon la revendication 1 ou 2,
**caractérisés en ce que**
seuls les points de coulée sont recouverts d'une couche d'or.

4. Eléments de construction selon la revendication 1 à 3,
**caractérisés en ce que**
la couche d'or présente une épaisseur comprise entre 3 et 100 µm.
